# FASCICULE DE BREVET EUROPEEN

(11) **EP 3 242 743 B1**
(45) Date de publication et mention de la délivrance du brevet: **06.11.2019**
(21) Numéro de dépôt: 15703307.7
(22) Date de dépôt: 06.01.2015
(51) Int. Cl.: A61K 8/81, B01J 13/00

(54) **PROCEDE DE SOIN OU MAQUILLAGE UTILISANT DES MICROGELS DE POLY(ETHYLENE GLYCOL-METHACRYLATE)**
SCHMINK- ODER PFLEGEVERFAHREN, WELCHES POLY(ETHYLENGLYKOL)METHACRYLATMIKROGELE VERWENDET
PROCESS OF CARE OR MAKE UP USING POLY(ETHYLENE GLYCOL) METHACRYLATE MICROGELS

(43) Date de publication de la demande: 15.11.2017
(60) Demande divisionnaire: 19191127.0
(73) Titulaire: LvmH Recherche, 45800 Saint-Jean De Braye (FR); Centre National de la Recherche Scientifique (C.N.R.S.), 75016 Paris (FR); UNIVERSITE DE PAU ET DES PAYS DE L'ADOUR, 64000 Pau (FR)
(72) Inventeur: TRANCHANT, Jean-François, F-45760 Marigny Les Usages (FR); GOMBART, Emilie, F-45100 Orleans (FR); BILLON, Laurent, F-64110 Saint Faust (FR); SAVE, Maud, F-65250 Escala (FR); BOULARAS, Mohamed, 78130 Les Mureaux (FR)
(74) Mandataire: Chantraine, Sylvie Hélène
(86) Numéro de dépôt international: PCT/FR2015/050019
(87) Numéro de publication internationale: WO 2016/110615

(56) Documents cités:
- WO-A1-2006/037901
- WO-A1-2006/037907
- MOHAMED BOULARAS ET AL: "Design of Smart Oligo(ethylene glycol)-Based Biocompatible Hybrid Microgels Loaded with Magnetic Nanoparticles", MACROMOLECULAR RAPID COMMUNICATIONS, vol. 36, no. 1, 25 novembre 2014 (2014-11-25), pages 79-83, XP055212999, ISSN: 1022-1336, DOI: 10.1002/marc.201400578 & MOHAMED BOULARAS ET AL: "Supporting informations, Design of Smart Oligo(ethylene glycol)-Based Biocompatible Hybrid Microgels Loaded with Magnetic Nanoparticles", MACROMOLECULAR RAPID COMMUNICATIONS, vol. 36, no. 1, 25 novembre 2014 (2014-11-25), pages 79-83, XP055213003, ISSN: 1022-1336, DOI: 10.1002/marc.201400578 & MOHAMED BOULARAS ET AL: "Supporting informations, Design of Smart Oligo(ethylene glycol)-Based Biocompatible Hybrid Microgels Loaded with Magnetic Nanoparticles", MACROMOLECULAR RAPID COMMUNICATIONS, vol. 36, no. 1, 25 novembre 2014 (2014-11-25), pages 79-83, XP055213003, ISSN: 1022-1

## Description

L'invention porte sur l'utilisation de microgels de poly(oligo-(éthylène glycol) méthacrylate) dans le domaine de la cosmétique.

Ces microgels présntent l'avantage d'être monodisperses, pH-sensibles et thermosensibles et de pouvoir incorporer des molécules organiques ou des particules inorganiques. Des films préparés à partir de solutions colloïdales de ces microgels chargés ou non en nanoparticules inorganiques présentent des propriétés optiques et électromécaniques très avantageuses.

### ART ANTERIEUR

Il existe plusieurs chimies de microgels thermosensibles. Les principales sont à base de poly(N-isopropylacrylamide) (PNIPAM), et plus rarement de poly(N-vinylcaprolactame) (PVCL) ou de poly(oligo-(éthylène glycol) méthacrylate).

Les différentes voies de synthèse de microgels poly(oligo-(éthylène glycol) méthacrylate) qui ont été décrites dans la littérature font intervenir des combinaisons de monomères tels le monomère di(éthylène glycol) méthyl éther méthacrylate (M(EO)₂MA, plutôt hydrophobe), et le monomère penta(éthylène glycol) méthyl éther méthacrylate (M(EO)₅MA plutôt hydrophile).

La synthèse d'un microgel thermosensible et pH-sensible, constitué d'un cœur thermosensible à base de P(M(EO)₂MA-*co*-M(EO)₅MA) et d'une écorce à base de mélange de poly(oligo-(éthylène glycol) méthacrylate) et de poly(acide acrylique) (P(M(EO)₂MA-*co*-M(EO)₅MA-*co*-AA)) a été décrite par Chi, C., T. Cai, and Z. Hu, Oligo(ethylene glycol)-Based Thermoresponsive Core-Shell Microgels. Langmuir, 2009. 25: p. 3814-3819. Ces microgels ont une structure cœur/écorce avec un cœur hydrophobe et une écorce hydrophile.

Il a été suggéré d'incorporer des molécules biologiquement actives dans des microgels à base de poly(oligo-(éthylène glycol) méthacrylate).

Par exemple un microgel thermosensible constitué d'un cœur poly(M(EO)₂MA) et d'une écorce poly(M(EO)₂MA-co-M(EO)₅MA) et des nanocapsules de poly(M(EO)₂MA-co-OEGMA) obtenues par greffage du polymère sur une particule de silice sacrificielle ont été proposés pour la délivrance de principe actif respectivement par Zhou, et al., Engineering oligo(ethylene glycol)-based thermosensitive microgels for drug delivery applications. Polymer, 2010. 51: p. 3926-3933 ; et par Wang, et al., Preparation of biocompatible nanocapsules with temperature-responsive and bioreducible properties, Journal of Materials Chemistry, 2011. 21: p. 15950.

Les microgels thermosensibles hybrides (ou nanocomposites) connus contenant des nanoparticules inorganiques sont des microgels à base de poly(N-isopropylacrylamide) (PNIPAM). Ces microgels présentent l'inconvénient de ne pas être biocompatibles.

Une première approche pour préparer ces matériaux consiste à synthétiser les microgels en présence des nanoparticules. Cette stratégie rend difficile la fabrication de ces microgels hybrides en raison de la complexité de la polymérisation. Le taux de nanoparticules incorporés est généralement faible et les microgels sont polydisperses. De plus, elle conduit généralement à une architecture de type cœur-écorce au sein de laquelle les nanoparticules sont réparties de manière uniforme.

Une seconde approche consiste à synthétiser, dans un premier temps, les microgels thermosensibles fonctionnalisés par des groupements ioniques. Par la suite, les nanoparticules inorganiques sont incorporées par co-précipitation des sels précurseurs des nanoparticules. La demande WO2004/081072 décrit par exemple des microgels PNIPAM présentant des unités anioniques acrylate de sodium (-COO⁻ Na⁺) et la co-précipitation *in situ* de sels précurseurs de nanoparticules diverses telles que des particules magnétiques (Fe₃O₄), des particules d'or (Au), et des particules Quantum dots (CdTe, CdS).

Une troisième approche consiste à incorporer les nanoparticules inorganiques par transfert de solvant. Des nanoparticules inorganiques hydrophobes sont synthétisées et dispersées dans une phase organique. Les microgels thermosensibles sont ajoutés dans la solution contenant les nanoparticules puis le tout est transféré en solution aqueuse, ce qui encapsule les nanoparticules dans les microgels. Cette méthode a fait l'objet d'un brevet en utilisant des microgels poly(N-isopropylacrylamide) et des nanoparticules de Quantum dots (CdS) pour des applications en photoluminescence (US 7914710).

Une quatrième et dernière approche consiste à synthétiser des microgels portant des charges ioniques et à faire adsorber des nanoparticules de charges opposées en surface du microgel. La préparation de latex poly(styrène-co-N-isopropylacrylamide) présentant des groupements cationiques 2-aminoéthyl méthacrylate (AEMA) a été décrite dans la demande WO 1997/045202. Des nanoparticules magnétiques γ-Fe₂O₃ chargées négativement en surface ont été adsorbées à la surface des latex par interaction électrostatique. Les nanoparticules ont ensuite été piégées dans la structure par synthèse d'une nouvelle écorce externe de PNIPAM à la surface des latex hybrides.

Plus récemment, des nanoparticules portant une charge positive (TiO₂) ont été incorporées dans une structure de microgel PNIPAM chargé par des unités comprenant un groupe acrylate (COO⁻) (US 8158005).

L'utilisation des microgels, notamment des microgels thermosensibles ayant des propriétés magnétiques, requiert dans certains domaines leur préparation sous forme de films minces.

La formation de films minces composés de microgels préalablement dispersés dans une phase aqueuse est un procédé de synthèse difficile car il nécessite de concilier deux facteurs opposés. Les microgels dispersés en phase aqueuse sont stabilisés par des charges répulsives qui empêchent les microgels de s'agréger ou de sédimenter. Or, les microgels doivent interagir les uns aux autres pour former des couches de microgels afin de former un film. Plusieurs procédés ont dû être développés pour les former.

Un premier procédé d'auto-assemblage de microgels sur surface modifiée consiste à ancrer des microgels à la surface d'un substrat préalablement traité pour créer des charges ioniques sur sa surface. Les microgels présentent des groupements ioniques issus généralement de l'amorceur de polymérisation ou d'un co-monomère ionique. Les microgels peuvent être ancrés à la surface d'un substrat par interaction électrostatique avec des groupements de charges opposées. Cette technique permet de déposer une couche mince de microgel sur le substrat (*monolayer technique*), mais il est également possible de multiplier ces couches par des traitements successifs de surface (*Layer-by-Layer technique*). Des microgels à base de poly(N-isopropylacrylamide-co-acide acrylique) ou P(NIPAM-*co*-AA) ont été déposés selon cette technique sur des substrats greffés par des groupements 3-aminopropyltriméthoxysilane (APTMS). Après chaque couche de microgel, un polymère présentant des charges positives (poly(allylamine hydrochloride) PAH ou poly(éthylène imine) PEI) a été ajouté sur le substrat modifié afin de régénérer les charges positives pour un pH inférieur à la constante d'acidité des amines.

Les films de microgels déposés sur substrat ont été utilisés pour la délivrance de principe actif tel que l'insuline pour le traitement du diabète (Nolan, C.M., M.J. Serpe, and L.A. Lyon, Thermally Modulated Insulin Release from Microgel Thin Films. Biomacromolecules, 2004. 5(5): p. 1940-1946) ou la doxorubicine pour le traitement de cellules cancéreuses (Serpe, M.J., et al., Doxorubicin Uptake and Release from Microgel Thin Films. Biomacromolecules, 2005. 6(1): p. 408-413).

Cette technique a l'avantage de contrôler les paramètres inhérents à l'élaboration de film mince de microgels (tels que l'épaisseur de film et la structuration des microgels.). L'inconvénient de ce procédé réside dans sa complexité d'élaboration qui nécessite d'utiliser des microgels particuliers (microgels chargés) et des substrats préalablement traités. Ceci empêche toute utilisation directe des microgels sur une surface quelconque.

Un second procédé d'élaboration d'un film d'hydrogel encapsulant des particules colloïdales consiste à encapsuler les particules dans un hydrogel afin de former un film souple et humide. Ce procédé est étudié principalement pour des applications photoniques. L'idée est d'associer les propriétés optiques de particules assemblées avec les propriétés mécaniques des hydrogels à l'état humide. Quelques exemples sont décrits dans la littérature et utilisent principalement des particules poly(styrène) sous forme de sphères dures. E. Tian et al. ont assemblé des particules de Poly(Styrene-co-Methacrylate de méthyl-co-acide acrylique) en plusieurs couches structurées et ont encapsulé le tout dans un hydrogel de poly(acrylamide) (Tian, E., et al., Colorful humidity sensitive photonic crystal hydrogel. Journal of Materials Chemistry, 2008. 18: p. 1116-1122). L'association des deux entités a permis de développer des hydrogels photoniques. Plus récemment, H. Jiang et al. ont également encapsulé des particules de poly(styrène) dans un hydrogel de poly(alcool vinylique) ou PVA (Jiang, H., et al., Photonic crystal pH and métal cation sensors based on poly(vinyl alcohol) hydrogel. New Journal of Chemistry, 2012. 36: p. 1051-1056.).

Plus récemment, H. Kim et al. ont assemblé des particules magnétiques d'oxyde de fer (Fe₃O₄) enrobé d'oxyde de silice (SiO₂) sous l'effet d'un champ magnétique et ont encapsulé cet assemblage dans un mélange de monomère poly(éthylène glycol) diacrylate et d'un photo-amorceur. La photopolymérisation du mélange permet de figer les particules dans une résine de poly(éthylène glycol acrylate). Les particules magnétiques figées dans la masse ont des propriétés photoniques pouvant être défini par le champ magnétique appliqué (US 2012/0028834). Cette technique présente l'avantage de produire des films de microgels plus souples car ces derniers sont supportés par un hydrogel « mou » en solution, et non un support solide. Cependant, la réalisation de ces films de particules reste complexe et différentes étapes de polymérisation sont nécessaires ce qui empêche tout auto-assemblage spontané lors d'une utilisation directe des particules.

Un troisième procédé de formation de films de microgels consiste à ajouter des fonctions réactives à la surface des microgels. Cette fonctionnalité est apportée par ajout d'un co-monomère lors de la synthèse des microgels. Ces fonctions réactives peuvent soit former des liaisons covalentes les unes avec les autres, soit former des liaisons covalentes par réaction avec une autre entité. Il est alors possible de former des nœuds de réticulation entre chaque microgels, le tout donnant un film composé de microgels liés chimiquement les uns aux autres. Ce procédé d'auto-assemblage est étudié principalement pour des applications photoniques. Le procédé d'auto-assemblage dépend du type de fonctionnalité ajoutée lors de la synthèse des microgels. Une grande majorité de ces travaux ont porté sur l'assemblage de microgel à base de PNIPAM.

Une première approche consiste à ajouter du poly(acide acrylique) (ou PAA) au sein de microgels de PNIPAM. L'auto-assemblage des microgels se fait grâce à des interactions faibles entre les fonctions acides carboxyliques du PAA. La somme des interactions permet d'auto-assembler les microgels et de gélifier le milieu.

Une seconde approche consistant à créer des liaisons covalentes a également été proposée, soit par ajout d'un réticulant dans la solution de microgels de PNIPAM, soit par polycondensation de microgels PNIPAM-co-NMA tel (NMA : N-methylol acrylamide ou N-hydroxymethyl acrylamide). Les microgels sont auto-assemblés par simple séchage d'une dispersion de microgels. La formation du film nécessite une étape supplémentaire de post-polymérisation thermique du réticulant ou de condensation thermique et acido-basique catalysée du NMA.

Une étude a utilisé des microgels de dérivé oligo-(éthylène glycol) méthacrylate présentant des fonctions polymérisables en surface. Ces microgels ont été réticulés par photo-polymérisation UV lors de leur assemblage pour des applications photoniques (US 2010/0076105). Cependant, la filmification requiert un temps de séchage très long d'environ quelques semaines.

Enfin, la préparation de microgels oligo (éthylène glycol) méthyl ether acrylate hybrides comprenant des nanoparticules magnétiques a été rapportée dans la publication Macromolecular Rapid Commun., 2015, 36, pages 79-83.

L'objectif de l'invention est de proposer un procédé cosmétique d'application sur la peau d'un microgel oligo-(éthylène glycol) méthacrylate qui présente au moins un des avantages suivants par rapport aux microgels de l'art antérieur : monodisperse ; pH-sensible ; biocompatible ; capable de former des microgels hybrides (ou nanocomposites) par adsorption de nanoparticules de charges opposées; capable de s'auto-assembler en plusieurs couches par simple procédé de séchage ; capable de former un film transparent ; capable de former un film cohésif et élastique ; sous forme d'un film, capable de générer un potentiel électrique par effet de compression ; sous forme d'un film, capable de diffracter la lumière générant ainsi une couleur.

Il a été découvert dans le cadre de la présente invention que certains poly(oligo-(éthylène glycol) méthacrylate) ont un fort potentiel filmogène par évaporation de l'eau à température ambiante. En particulier, l'invention propose pour la première fois l'auto-assemblage de microgels hybrides magnétiques sous forme de film mince possédant des propriétés de structuration.

Les films de microgels obtenus selon le procédé de l'invention présentent l'avantage d'être totalement auto-supportés. Les microgels n'étant pas encapsulés ni supportés, lors de la filmification l'interaction entre les microgels et le substrat sur lesquels ils sont déposés, par exemple la peau, est maximale. Les films sont obtenus par simple séchage à température ambiante et aucun amorceur radicalaire n'est nécessaire. Les microgels contenant ou non des nanoparticules sont capables de s'auto-assembler en plusieurs couches pour former un film transparent.

Il a également été découvert dans le cadre de la présente invention que les microgels poly(oligo-(éthylène glycol) méthacrylate) sont des matériaux polyélectrolytes capables de générer un champ électrique par action mécanique.

### DESCRIPTION DE L'INVENTION

L'invention porte sur un procédé cosmétique de soin ou de maquillage comprenant l'application sur la peau de microgels poly(oligo-(éthylène glycol) méthacrylate) dotés de propriétés colloïdales ainsi que d'une sensibilité aux variations de température et/ou de pH dans l'eau, grâce à la présence de groupements -COOH éventuellement salifiés.

On entend par « microgel » au sens de l'invention, un polymère réticulé sous forme d'une particule sphérique de taille variant de 100 nm à 500 nm à l'état sec (c'est-à-dire contenant moins de 2% en poids d'eau), de préférence entre 350 et 450 nm, de préférence encore de l'ordre de 400 nm. Le microgel est un microhydrogel en ce sens qu'il est susceptible d'être obtenu par un procédé de copolymérisation en phase aqueuse de plusieurs monomères. Le microgel n'a pas une structure cœur/écorce : les monomères qui le composent sont répartis de façon uniforme sur l'ensemble du volume de la particule, ce qui lui confère des propriétés particulières.

Les microgels utilisés dans le procédé de l'invention peuvent présenter l'avantage d'être à la fois monodisperses, thermosensibles, pH-sensibles et biocompatibles. Les microgels peuvent être à la fois thermosensibles et biocompatibles, contrairement aux microgels thermosensibles de l'art antérieur qui sont généralement des structures à base de poly(alkylacrylamide).

Ces microgels présentent l'originalité de comprendre un mélange d'unités répétitives d'oxyde d'éthylène branchés et d'unités comprenant un groupement acide carboxylique (-COOH) ou carboxylate (-COO⁻) dont on peut faire varier la teneur en fonction des applications visées. Ces groupements confèrent aux microgels la propriété de pH-sensibilité.
L'invention a ainsi pour objet un procédé de soin ou de maquillage cosmétique qui consiste à appliquer sur la peau des microgels ou un produit cosmétique contenant lesdits microgels,
les microgels étant susceptibles d'être obtenus par polymérisation par précipitation en phase aqueuse en présence d'un agent réticulant, des trois monomères suivants :
- le di(éthylène glycol) méthyl éther méthacrylate (M(EO)₂MA),
- un oligo(éthylène glycol) méthyl éther méthacrylate (M(EO)ₙMA) n étant un entier allant 3 à 12,
- un monomère de formule CR₁R₂=CR₃R₄ dans laquelle R₁, R₂, R₃ et R₄ représentent un hydrogène, un halogène ou un groupement hydrocarboné, à la condition qu'au moins un des quatre groupements comprenne un groupement -COOH ou -COO⁻M⁺, tel que M⁺ représente un cation,
   et ledit produit cosmétique contenant, outre les microgels, au moins un composé choisi dans le groupe constitué par les agents tensio-actifs, les huiles, les produits biologiquement actifs, les pigments et les colorants.

Leurs propriétés thermosensibles et pH-sensibles ont également été démontrées et décrites dans ce document.

Dans la suite du texte, on désigne sous le terme « -COOH », la forme acide - COOH ou salifiée -COO⁻M⁺, pour alléger l'écriture.

M(EO)₂MA représente par exemple 50 à 90% en moles du nombre total de moles des monomères, M(EO)ₙMA représente de préférence 10 à 50% en moles du nombre total de moles des monomères, le monomère de formule CR₁R₂=CR₃R₄ représente de préférence 0,1 à 20 % en moles du nombre total de moles des monomères, la somme de ces trois teneurs étant égale à 100%.

La ratio molaire entre M(EO)₂MA et M(EO)ₙMA est de préférence compris entre 1 :1 et 20 :1, par exemple entre 5 :1 à 10 :1. L'expression « compris entre » au sens de l'invention exclut les bornes numériques qui lui succèdent. En revanche, l'expression « allant de ...à » vise à inclure les bornes exprimées.

Le nombre de moles de monomère de formule CR₁R₂=CR₃R₄ peut être compris entre 0 et 20 mol%, par exemple aller de 0,1 à 5 mol % du nombre total de moles totale des trois monomères.

Selon un mode de réalisation, M(EO)₂MA représente par exemple 80 à 90% en moles du nombre total de moles des trois monomères, M(EO)ₙMA représente de préférence 5 à 15% en moles du nombre total de moles des monomères et l'acide méthacrylique représente de préférence 0,1 à 10 % en moles du nombre total de moles des monomères, la somme de ces trois teneurs étant égale à 100%.

Le monomère de formule CR₁R₂=CR₃R₄ est de préférence tel que R₁ et R₂, représentent chacun un hydrogène, R₃ représente H ou un groupement alkyle, de préférence en C1-C6, éventuellement substitué à -OH ou -COOH, et R₄ représente indépendamment de R₃ le groupement-COOH ou un groupement alkyle, de préférence en C1-C6, éventuellement substitué à -OH ou -COOH. Le groupement alkyle peut être méthyle, éthyle ou n-butyle. Selon un mode de mise en œuvre particulier, R₁ et R₂, représentent chacun un hydrogène et R₃ et R₄ représentent indépendamment -H, - COOH, ou -CH₂-COOH.

Le monomère de formule CR₁R₂=CR₃R₄ peut être par exemple choisi parmi les acides méthyl acrylique, méthyl méthacrylique, éthyl acrylique, éthyl méthacrylique, n-butyl acrylique, n-butyl méthacrylique.

Selon un mode de réalisation, le monomère de formule CR₁R₂=CR₃R₄ peut être l'acide méthacrylique ou l'acide itaconique. L'acide acrylique peut être exclu de la définition du monomère de formule CR₁R₂=CR₃R₄ dans certains cas.

L'agent réticulant peut être choisi dans le groupe constitué par les oligo(éthylène glycol) diacrylate comprenant de 1 à 10 motifs éthylène glycol, 1,3-Butanediol diacrylate, 1,4-Butanediol diacrylate, 1,6-Hexanediol diacrylate, Pentaerythritol diacrylate monostearate, Glycerol 1,3-diglycerolate diacrylate, Neopentyl glycol diacrylate, Poly(propylene glycol) diacrylate, 1,6-Hexanediol éthoxylate diacrylate, Trimethylolpropane benzoate diacrylate, éthylène glycol diméthacrylate, 1,3-Butanediol diméthacrylate, 1,4-Butanediol diméthacrylate, 1,6-Hexanediol dimethacrylate, glycerol dimethacrylate, N,N divinylbenzene, N,N-Methylenebisacrylamide, N,N-(1,2-Dihydroxyethylene)bisacrylamide, Poly(ethylene glycol) diacrylamide, Allyl disulfide, Bis(2-methacryloyl)oxyethyl disulfide et N,N-Bis(acryloyl)cystamine.

L'agent réticulant représente par exemple de 1 à 5 % en moles du nombre total de moles des trois monomères.

Les monomères utilisés sont de préférence le di(ethylene glycol) methyl ether methacrylate (M(EO)₂MA, Mn 250 g.mol⁻¹), l'oligo(ethylene glycol) methyl ether methacrylate (M(EO)₉MA, Mn 475 g.mol⁻¹), l'acide méthacrylique (MAA).

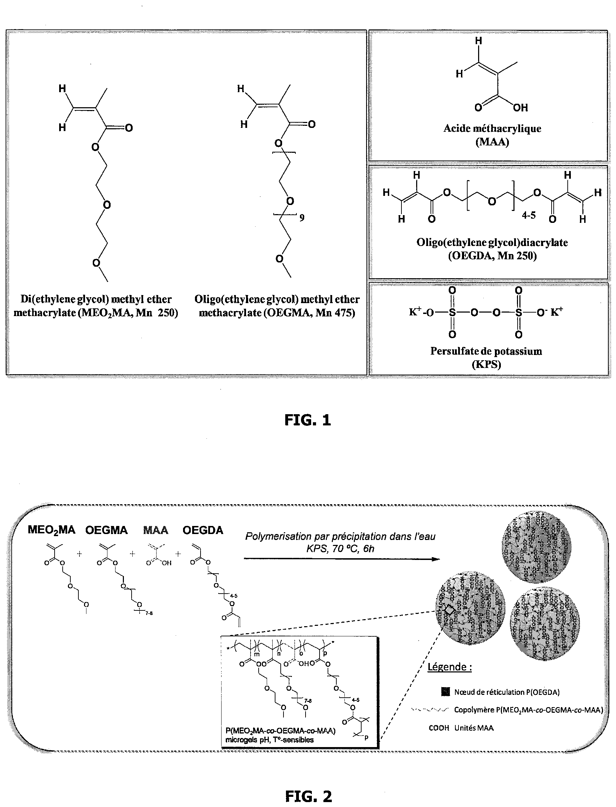

L'agent réticulant est par exemple l'oligo(ethylene glycol)diacrylate comprenant 4 à 5 unités oxyde d'éthylène (OEGDA, Mn 250 g.mol⁻¹). Les structures chimiques des monomères et de l'agent réticulant préférés sont représentées sur La Figure 1.

La taille moyenne d'un microgel peut varier selon qu'il est sec ou en solution aqueuse : ainsi un microgel à l'état sec peut atteindre quatre fois sa taille initiale lorsqu'il est placé en solution aqueuse à 20°C. La taille moyenne d'un microgel à l'état sec peut aller de 100 à 1000 nm. La fonction de distribution radiale hydrodynamiques des microgels mesurée à un angle de 60° et à une température de 20°C, est avantageusement inférieure à 1,1, ce qui confère aux microgels la qualité de monodisperses.

Les microgels peuvent comprendre des particules organiques ou inorganiques : on les nomme couramment dans ce cas des microgels hybrides. Les particules introduites ont de préférence une taille comprise entre 1 et 150 nm, par exemple entre 5 et 50 nm, et sont nommées des nanoparticules. Les nanoparticules peuvent être magnétiques ou non.

Les microgels peuvent être des microgels hybrides monodisperses, thermosensibles et magnétiques à base de poly(oligo-(éthylène glycol) méthacrylate) contenant des nanoparticules magnétiques.

Certains microgels hybrides monodisperses, thermosensibles et magnétiques à base de poly(oligo-(éthylène glycol) méthacrylate) sont obtenus à partir d'au moins deux monomères
- le di(éthylène glycol) méthyl éther méthacrylate (M(EO)₂MA), et
- un oligo(éthylène glycol) méthyl éther méthacrylate (M(EO)ₙMA) n étant un entier allant 3 à 12, et éventuellement en présence d'un troisième monomère :
- un monomère de formule CR₁R₂=CR₃R₄ dans laquelle R₁, R₂, R₃ et R₄ représentent un hydrogène, un halogène ou un groupement hydrocarboné, à la condition qu'au moins un des quatre groupements comprenne un groupement -COOH ou -COO⁻M⁺, tel que M⁺ représente un cation.

Un procédé de préparation des microgels hybrides monodisperses, thermosensibles et magnétiques à base de poly(oligo-(éthylène glycol) méthacrylate) consiste à :
- préparer une dispersion colloïdale de nanoparticules chargées positivement en surface et placées en solution aqueuse,
- préparer une dispersion colloïdale aqueuse de microgels selon l'une des revendications 1 à 4,
- mélanger les deux dispersions colloïdales et ajuster du pH au-dessus du point isoélectrique des nanoparticules.

Selon un mode de réalisation, les nanoparticules sont chargées positivement à leur surface lorsqu'elles sont placées en solution aqueuse si bien que les microgels hydrides peuvent être préparés par simple mélange de deux dispersions colloïdales : une première dispersion colloïdale de microgels et une deuxième dispersion colloïdale de nanoparticules. Les paramètres clés permettant la réussite de ce procédé résident, d'une part, dans l'ajout de groupements carboxyliques ou carboxylates répartis de manière homogène au sein du microgel et, d'autre part, dans la charge positive de surface des nanoparticules. Le tout permet d'encapsuler de manière contrôlée les nanoparticules au sein du microgel, tout en conservant les propriétés colloïdales et thermosensibles du matériau final. L'architecture hybride des microgels ainsi que leurs propriétés thermosensibles sont démontrées dans les exemples. L'incorporation des nanoparticules au sein des microgels est tout d'abord démontrée avec un taux important et quantitatif de nanoparticules magnétiques encapsulés (taux de charge testés allant de 0 à 33 % massique de nanoparticules par microgel hybride). Les propriétés thermosensibles en solution aqueuse des microgels hybrides sont également démontrées quelques soit le taux de charge en nanoparticules magnétiques.

Selon un mode de réalisation, les nanoparticules sont des pigments, des colorants ou des filtres solaires couramment utilisés dans le domaine de l'optique, de la cosmétique, de l'agroalimentaire ou de la pharmacie.

Les particules peuvent comprendre au moins un métal ou un oxyde métallique. Le métal peut être l'or, l'argent, l'étain, le titane, le cuivre ou l'aluminium. L'oxyde métallique peut être choisi dans le groupe constitué par les oxydes de fer, de titane, de zinc, de chrome et d'étain. Les particules comprennent par exemple au moins un des composés suivants: TiO₂, Fe₂O₃, TiFe₂O₅, Ti-suboxydes, Fe₃O₄, Cr₂O₃, ZrO₂, ZnO, SnO₂, Sn(Sb)O₂.

Selon un mode de réalisation, les nanoparticules sont des nanoparticules magnétiques d'oxyde de fer (Fe₂O₃, maghémite) de taille comprise entre 1 et 150 nm, par exemple entre 6 et 30 nm.

Les nanoparticules magnétiques peuvent être synthétisées par co-précipitation de sels métalliques (Fe²⁺ et Fe³⁺) en phase aqueuse, puis oxydation afin de produire des nanoparticules magnétiques (γ-Fe₂O₃) stabilisées en solution par des charges positives. Le procédé de synthèse des nanoparticules de maghémites peut être celui développé par Massart, R., Préparation of aqueous magnetic liquids in alkaline and acidic media. IEEE Trans. Magn., 1981. 17(2): p. 1247-1248.

La préparation de microgels hybrides thermosensibles à base de poly(oligo-(éthylène glycol) méthacrylate) et de nanoparticules peut être réalisée préférence par un procédé de mélange des deux constituants sous forme de dispersions colloïdales. Les nanoparticules placées en solution aqueuse sont susceptibles d'être chargées positivement en surface lorsqu'elles sont mises en contact avec les microgels. La stabilité des microgels hybrides est obtenue par une augmentation de pH au-dessus du point isoélectrique des nanoparticules inorganiques tout en préservant l'encapsulation des nanoparticules.

Ce procédé présente l'avantage d'être simple de mise en œuvre. La répartition homogène des groupements acides carboxyliques ou carboxylate au sein du microgel et, le choix de nanoparticules chargées positivement en surface permet d'encapsuler de manière contrôlée les nanoparticules au sein du microgel, tout en conservant les propriétés colloïdales et thermosensibles du matériau final.

Les microgels hybrides peuvent contenir jusqu'à 50% en poids, notamment jusqu'à 35% en poids de nanoparticules sans perdre leurs propriétés colloïdales, pH et thermosensibles. Le taux de nanoparticules par microgels hybrides peut être déterminé par analyse thermogravimétrique (TGA).

Les microgels décrits précédemment comprenant éventuellement des nanoparticules sont capables de s'auto-assembler pour former un film constitué d'une ou de plusieurs couches de microgels, par un procédé de séchage ou d'évaporation d'une suspension aqueuse desdits microgels.

Les films formés sont cohésifs et élastiques. Les microgels peuvent ainsi être utilisés comme agent filmogène dans des compositions cosmétiques, de manière à améliorer la tenue de ces compositions sur les matières kératiniques. Après séchage, les films ne se re-dispersent pas lorsqu'ils sont immergés dans l'eau

Les microgels et les films qu'ils forment peuvent générer un potentiel électrique par effet de compression (effet Donan). Les films sont préparés à partir de microgels comportant des sites ioniques issus des fonctions carboxylate (COO⁻). Ces sites ioniques sont contraints dans la structure et créent des polarisations au sein du microgel (e.g. microgels polyélectrolytes). Lorsque le film est soumis à une pression, un mouvement des contre-ions contribue à créer une polarisation au sein même du film ce qui génère une différence de potentiel électrique entre la surface et la masse du film. Les inventeurs ont trouvé que la présence d'acide méthacrylique améliore les propriétés mécano-électriques des films.

L'auto-assemblage des microgels monodisperses permet également une diffraction de la lumière générant ainsi une couleur. Ces propriétés photoniques peuvent être modulées par la composition même des microgels.

Les microgels monodisperses de l'invention peuvent s'auto-assembler de manière périodique sous forme de cristaux colloïdaux. Cet auto-assemblage particulier permet la diffraction d'une lumière incidente et génère ainsi une couleur observable en fonction de l'angle d'observation. Cet effet varie en fonction de la composition des microgels : **a)** le séchage d'une dispersion de microgels sans nanoparticules induit la formation d'un film sec totalement transparent, incolore et ne diffractant pas la lumière. Alors que ce même film diffracte la lumière à l'état humide avec des couleurs observables en solution. **b)** Le séchage d'une dispersion de microgels contenant des nanoparticules magnétiques (microgels hybrides) induit la formation d'un film sec transparent, coloré (marron) et diffractant la lumière. Dès lors, le film est marron à un angle d'observation de 90°C (couleur issu des nanoparticules magnétiques) et change de couleur en réflexion à des angles d'observation plus faibles.

La présence de nanoparticules magnétiques au sein des microgels permet d'orienter les microgels à l'aide d'un aimant permanent et d'améliorer les propriétés mécaniques des films. Au cours du séchage sur une surface donnée, les microgels thermosensibles et magnétiques peuvent être guidés et concentrés en un point précis à l'aide d'un aimant permanent. Cela a pour effet de faire varier l'épaisseur et la couleur des films (teinte marron plus ou moins forte). De plus les nanoparticules magnétiques améliorent les propriétés mécaniques des films en milieu humide permettant ainsi d'exercer de plus forte compression sur le film.

L'ensemble de ces propriétés permet d'envisager l'utilisation des microgels de l'invention et des films qu'ils forment pour la mise en œuvre d'un procédé cosmétique de soin ou de maquillage de la peau. Ces produits peuvent stimuler la peau en générant un courant électrique et éventuellement délivrer une molécule biologiquement active par effet de compression. La molécule biologiquement active peut être encapsulée dans les microgels ou être présente dans le produit

Un produit cosmétique peut être constitué ou contenir des microgels tels que décrits précédemment et éventuellement au moins un composé choisi dans le groupe constitué par les agents tensio-actifs, les huiles, les produits biologiquement actifs, les pigments et les colorants.

Le microgel peut contenir toutes sortes d'ingrédients ou d'excipients utilisés dans le domaine cosmétique et pharmaceutique, de préférence des pigments à base d'oxyde de fer ou des substances biologiquement actives.

Les microgels peuvent être synthétisés par un procédé de polymérisation par précipitation, à partir des monomères préalablement solubilisés en solution aqueuse.

Un procédé de polymérisation par précipitation d'un microgel de poly(éthylène glycol-méthacrylate) tel que décrit précédemment, peut comprendre une étape de mise en contact en phase aqueuse en présence d'un agent réticulant des trois monomères décrits précédemment, à une température comprise entre 40 et 90°C. Le procédé ne requiert pas la présence d'un agent tensio-actif tel que le SDS (dodecyl sulfate sodium).

La polymérisation des monomères peut être amorcée par ajout d'un amorceur radicalaire hydrosoluble, par exemple le persulfate de potassium (KPS) à une température comprise entre 40 et 90°C, de préférence de l'ordre de 70°C.

On préfère ajouter une solution aqueuse du monomère de formule CR₁R₂=CR₃R₄ de façon graduelle dans une solution aqueuse des deux autres monomères, de manière à garantir la répartition homogène des groupements -COOH dans les microgels qui précipitent.

A la température de polymérisation, le polymère formé est hydrophobe et précipite dans le milieu réactionnel aqueux sous forme de particules sphériques, la présence du réticulant OEGDA au cours de la polymérisation permet de figer les particules sous cette forme sphérique en créant des nœuds de réticulation.

Les microgels présentent l'avantage d'être de structure homogène, car les groupements -COOH et les nœuds de réticulation sont répartis de façon régulière sur tout leur volume.

Les films de microgels sont avantageusement préparés par un procédé de séchage ou d'évaporation de solvant à 20°C, par exemple en partant d'une dispersion colloïdale de microgels monodisperses à une concentration massique pouvant varier de 1.4 à 5 wt% dans l'eau.

Selon ce procédé, au moins un premier volume de solution peut être laissé sécher jusqu'à complète évaporation de l'eau à température ambiante. Cette étape peut être répétée plusieurs fois pour obtenir un film composé de plusieurs couches de microgels monodisperses et d'épaisseur pouvant varier entre 350 et 450 micromètres à l'état sec.

Un kit comprenant un aimant et un produit cosmétique contenant des microgels décrit précédemment et comprenant des nanoparticules magnétiques, l'aimant et le produit étant conditionnés ensemble peut être utilisé dans la mise en œuvre du procédé de soin ou de maquillage de l'invention.

### BREVE DESCRIPTION DES FIGURES

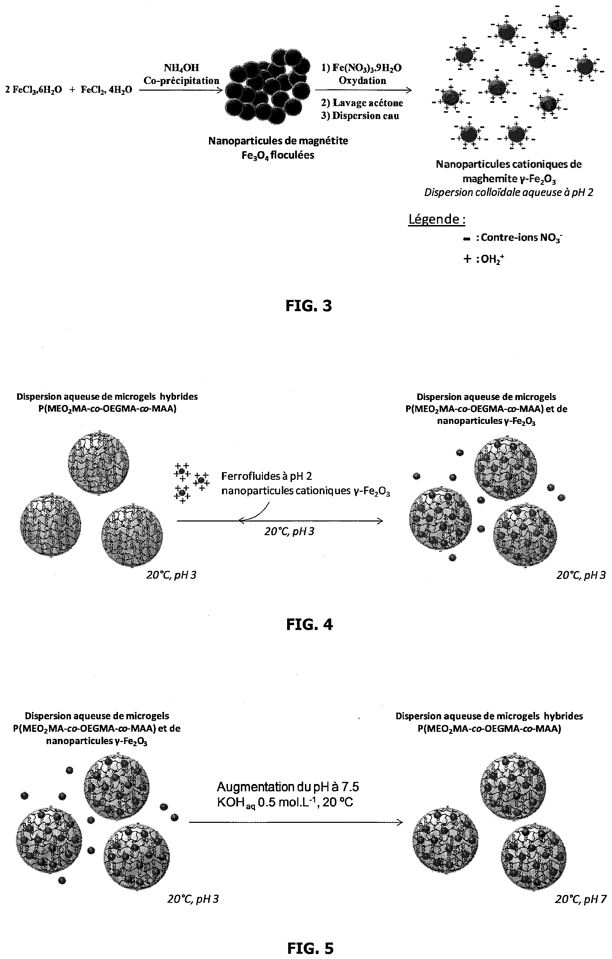

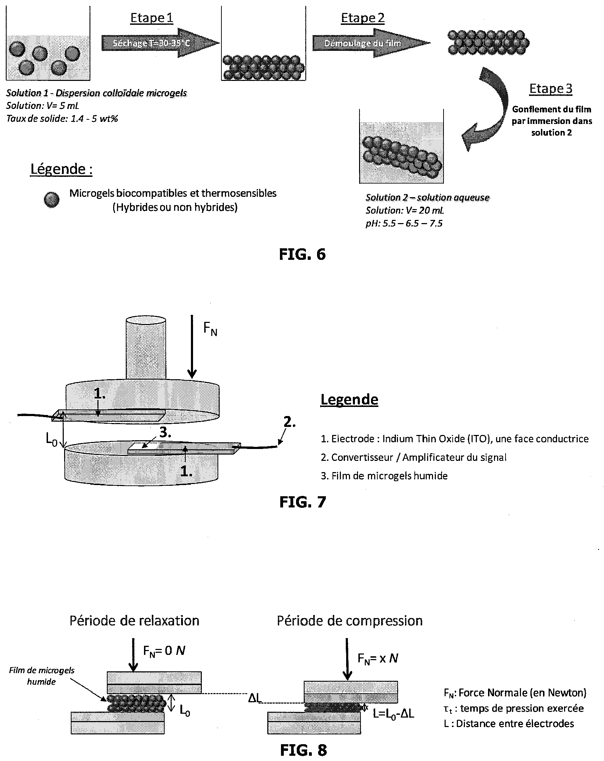

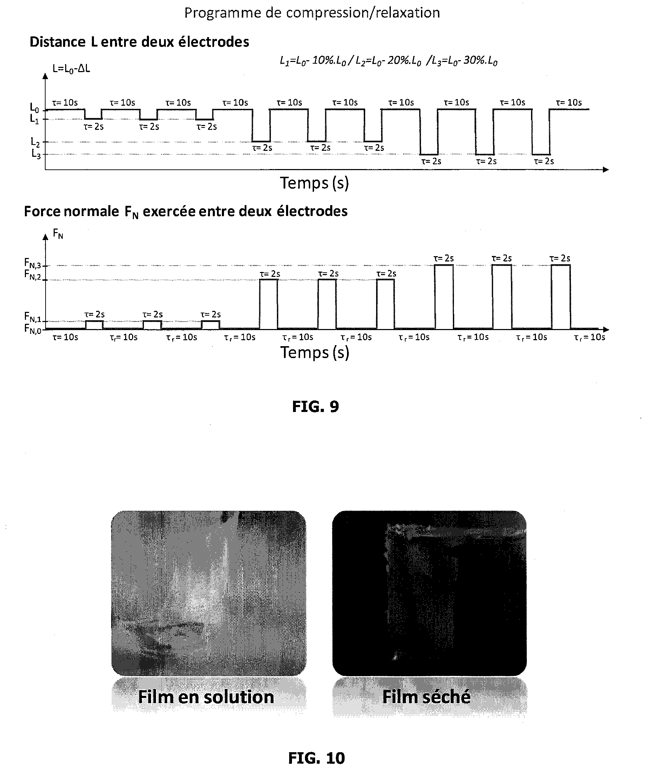

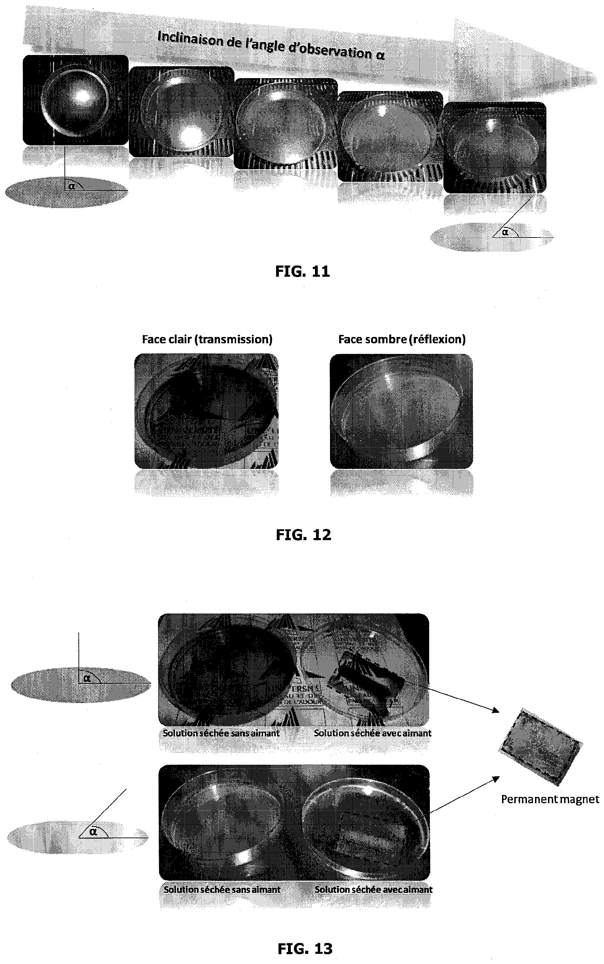

**La** **Figure 1** présente les structures chimiques des monomères utilisés pour la synthèse de microgels biocompatibles.
**La** **Figure 2** est un schéma de synthèse de microgels biocompatibles pH-sensibles et thermo-sensibles à base de poly(MEO₂MA-*co*-OEGMA-*co*-MAA).
**La** **Figure 3** est un schéma de préparation de particules de γ-Fe₂O₃.
**Les** **Figures 4 et 5** sont des représentations schématiques respectivement de la 1^{ère} et de la 2^{eme} étape de synthèse du procédé de préparation de microgels hybrides contenant des particules de γ-Fe₂O₃.
**La** **Figure 6** représente un schéma du procédé de formation des films de microgels.
**La** **Figure 7** représente un montage de caractérisation de l'effet mécano-électrique des films de microgels.
**La** **Figure 8** est une représentation schématique de la période de compression et de relaxation d'un film de microgels.
**La** **Figure 9** représente le schéma d'un programme de compression et de relaxation de microgels.
**La** **Figure 10** est un cliché de films de microgels à l'état sec et gonflé en solution.
**La** **Figure 11** est un cliché d'un film sec de microgels hybrides contenant des nanoparticules de γ-Fe₂O₃ pris à différents angles d'observations.
**La** **Figure 12** est un cliché d'un film sec de microgels hybrides contenant des nanoparticules de γ-Fe₂O₃ pris sur une surface clair et sur une surface sombre.
**La** **Figure 13** est un cliché d'un film sec de microgels hybrides contenant des nanoparticules de γ-Fe₂O₃ avec et sans aimant.

L'invention est également illustrée par les exemples suivants.

### EXEMPLE 1 : Synthèse d'un microgel à base de poly(oligo-(éthylène glycol)méthacrylate)

On a utilisé les monomères suivants : di(éthylène glycol) méthyl éther méthacrylate (M(EO)₂MA, Mn 250 g.mol⁻¹), oligo(éthylène glycol) méthyl éther méthacrylate (M(EO)₄₋₅MA encore noté OEGMA dans la suite, Mn 475 g.mol⁻¹), et acide méthacrylique (MAA). L'agent de réticulation était l'oligo(éthylène glycol)diacrylate (OEGDA, Mn 250 g.mol⁻¹).

### Protocole expérimental:

0.966 g de MEO₂MA (5.14x10⁻³ mol), 0.272 g d'OEGMA (5.73x10⁻⁴ mol) et 0.029 g de OEGDA (1.17x10⁻⁴ mol) sont introduits dans volume de 57.5 mL d'eau et laissés sous agitation magnétique jusqu'à complète dissolution des monomères. Le mélange est alors filtré et introduit dans un tricol d'un volume de 250 mL équipé d'un agitateur mécanique avant d'être dégazés à l'azote durant 45 min sous agitation mécanique (150 rpm). Une solution aqueuse de MAA (0.026 g, 3.05x10⁻⁴ mol dissous dans 2 mL d'eau) est alors introduite dans le milieu réactionnel. Le mélange est laissé à 70°C durant 20 min avant d'introduire une solution aqueuse de persulfate de potassium (KPS, 0.0143 g dissout dans 2.5 mL d'eau) préalablement dégazé à l'azote. L'ajout de KPS permet d'amorcer la polymérisation et le milieu réactionnel est laissé sous agitation mécanique (50 rpm) à 70°C durant 6h.

La polymérisation est ensuite arrêtée par ajout d'oxygène et laissée refroidir jusqu'à température ambiante. Les microgels sont alors séparés du milieu réactionnel par centrifugation (10 000 rpm, 30 min) et le milieu réactionnel est remplacé par de l'eau pure (de grade milliQ), l'étape est répétée cinq fois.

La solution finale se compose alors d'une dispersion colloïdale de microgels P(MEO₂MA-*c*o-OEGMA-*co*-MAA) en phase aqueuse, cette dispersion est gardée à température ambiante.

### Propriété du microgel

La synthèse des microgels a été caractérisée par suivi cinétique des monomères en utilisant la spectroscopie à résonance magnétique nucléaire du proton (RMN ¹H). On observe une conversion totale des monomères ainsi qu'une composition homogène des microgels avec une distribution homogène des nœuds de réticulation et des unités acide méthacrylique.

Le rendement final en microgel réticulé a été analysé par extrait sec du milieu réactionnel et permet de déterminer un rendement de 70 % massique en microgel réticulé.

Le taux d'acide méthacrylique incorporé a été déterminé par dosage acido-basique des microgels purifiés. Le caractère pH-sensible des microgels en solution aqueuse ainsi que l'incorporation de 70% molaire du monomère MAA initial a pu être vérifiée.

Les microgels ont été observés par microscopie électronique à transmission (MET) et leurs tailles déterminées par diffusion dynamique de la lumière. Les microgels observés sont monodisperses, avec une taille de 400 nm à l'état séché et pouvant aller jusqu'à 1000 nm à l'état humide.

### EXEMPLE 2 : Préparation de solutions de microgel à base de poly(oligo-(éthylène glycol)méthacrylate)

1.2 mL d'une solution de microgels préparés selon l'Exemple 1 (à 15 g.L⁻¹ de microgels) est dispersé dans 10 mL d'une solution d'eau pure (grade milliQ). Le pH de la dispersion est ajusté par ajout d'une solution d'acide chlorhydrique ou d'hydroxyde de potassium à 0.1 mol.L⁻¹. Le mélange est laissé sous agitation magnétique jusqu'à stabilisation du pH. La taille des microgels en solution est mesurée par diffusion dynamique de la lumière et la température de solution est contrôlée au cours de l'analyse.

En étudiant la taille des microgels en solution par diffusion dynamique de la lumière, il a été possible d'évaluer l'impact du pH et de la température du milieu sur la capacité des microgels à gonfler ou à se contracter dans l'eau.

Les microgels sont sensibles aux variations de pH du milieu passant d'une taille de 400 nm à pH < 5.5 à une taille de 1000 nm à pH > 6.0.

Les microgels sont thermosensibles et passent d'un état gonflé à 20°C à un état contracté à température supérieure. La température de contraction est fonction du pH (35°C à pH < 6.0 et 55°C à pH > 7.0). Enfin, le volume du microgel gonflé à 20°C diminue jusqu'à 3 fois par rapport à son volume initial lorsque la température passe au-delà de la température de contraction.

### EXEMPLE 3 : Préparation d'un microael à base de poly(oligo-(éthylène glycol)méthacrylate) contenant des nanoparticules magnétiques

### Synthèse des nanoparticules de maghémites γ-Fe₂O₃.

Les réactifs utilisés sont : le chlorure ferreux tétrahydraté (FeCl₂.4H₂O), le chlorure ferrique hexahydraté (FeCl₃, 6H₂O), l'hydroxyde d'ammonium (NH₄OH) à 28-30% w/w, le nitrate de fer (Fe^{III}(NO₃)₃.9H_{z}O), l'acide chlorhydrique (HCl) à 36 % v/v et l'acide nitrique (HNO₃).

Les nanoparticules de maghémite utilisées lors de cette étude ont été synthétisées par co-précipitation des sels métalliques (Fe^{II} et Fe^{III}). Cette méthode de synthèse consiste à former des nanoparticules de magnétite (Fe₃O₄) par co-précipitation de chlorure ferreux (FeCl₂) et de chlorure ferrique (FeCl₃) en milieu basique par ajout d'hydroxyde d'ammonium (NH₄OH). La magnétite est ensuite oxydée pour former la variété maghémite (γ-Fe₂O₃). L'oxydation de la magnétite en maghémite permet d'établir des fonctions hydroxyles sensibles au pH à la surface des nanoparticules, ces fonctions présentent un point de charge nulle à un pH neutre (pH ≈ 7.2). Ainsi, à des pH acides ou basiques, ces nanoparticules présentent un état colloïdal en phase aqueuse par répulsion électrostatique de charges anioniques (à pH basique) ou cationiques (à pH acide). Il s'agit là d'une méthode de synthèse versatile de nanoparticules magnétiques stabilisées en phase aqueuse, communément appelées « ferrofluides cationiques » ou « ferrofluides anioniques » selon le pH de stabilisation.

### Protocole expérimental

### Etape 1 : Formation de la magnétite

12.2 g de chlorure ferrique hexahydrate FeCl_{3.}6H₂O (0,0451 mol) sont introduits dans un bécher de 3 L contenant 500 mL d'eau pure. 4.49 g de Chlorure ferreux tetrahydrate FeCl₂.4H₂O (0,0226 mol) sont dissouts dans 24.3 mL d'une solution d'acide chlorhydrique (HCl) à 1,5 mol.L⁻¹ est ajouté dans le bécher de 3 L et le tout est laissé mélangé sous faible agitation mécanique (ratio initial Fe^{II}/Fe^{III}=0,5). Un volume V= 43 mL d'hydroxyde d'ammonium à 28/30% w/w est alors ajouté au bécher sous forte agitation mécanique et à température ambiante. L'ajout d'hydroxyde d'ammonium mène à la formation de magnétites (Fe₃O₄) floculées en solution aqueuse basique (pH > 10), les floculats de magnétite sont alors laissés décanter sous l'effet d'une attraction magnétique générée par un aimant permanent puis le surnageant est éliminé et remplacé par de l'eau pure (grade milliQ). L'étape de lavage est répétée deux fois afin d'éliminer l'excès d'hydroxyde d'ammonium.

### Etape 2 : Désorption des contre-ions ammonium et oxydation de surface.

Après les étapes successives de lavage de la magnétite, un volume V= 28.6 mL d'une solution aqueuse d'acide nitrique HNO₃ à 2 mol.L⁻¹ est ajouté aux floculats de magnétite et est laissé sous agitation mécanique durant 30 min afin de traiter la surface des particules de magnétite.

L'ajout d'acide nitrique permet d'acidifier le milieu et d'induire une désorption des contre-ions ammonium NH₄⁺ en excès à la surface des nanoparticules par échange ionique avec les ions nitrate NO₃⁻. L'oxydation des particules en surface permet également de solubiliser les ions ferreux n'ayant pas précipités et présents en surface des nanoparticules.

Les floculats de magnétite traités en surface sont laissés décanter sous un aimant permanent puis le surnageant est éliminé et remplacé par de l'eau pure, cette étape est répétée deux fois.

### Etape 3 : Oxydation du cœur des nanoparticules.

Après les étapes successives de lavage des magnétites traitées en surface, un volume V= 85.7 mL d'une solution fraîchement préparée de nitrate ferrique Fe^{III}(NO₃)_{3.9}H₂O à 0.33 mol.L⁻¹ est ajoutée à ébullition aux floculats de magnétite et est laissée à reflux et sous agitation mécanique durant 45 min.

L'apport des ions Fe³⁺ par le nitrate ferrique permet d'oxyder les Fe^{II} des particules formant ainsi la variété maghémite γ-Fe₂O₃. Après oxydation totale des particules, le floculat de maghémite est laissé décanter sous aimant permanent et le surnageant est éliminé puis remplacé par de l'eau pure, l'opération est répétée deux fois.

### Etape 4 : « Peptisation » des nanoparticules de magnétite.

Un volume V= 28.6 mL d'une solution d'acide nitrique HNO₃ à 2 mol.L⁻¹ est ajouté au floculat de maghémite et laissé à température ambiante et sous agitation mécanique durant 30 min. L'ajout d'acide nitrique permet d'apporter des ions hydronium H⁺ en surface de la maghémite. Le floculat de maghémite cationique est laissé décanter puis lavé trois fois à l'acétone. Un volume V= 70 mL d'eau est alors ajouté aux nanoparticules permettant une « *peptisation* » des nanoparticules dans l'eau, la dispersion de nanoparticule est alors stabilisée par répulsion électrostatique de charges positives à la surface des nanoparticules. Enfin l'acétone résiduelle est éliminée par évaporation sous vide à 40°C.

### Synthèse des microgels hybrides P(MEO₂MA-co-OEGMA-co-MAA) / v-Fe₂O₃.

Les microgels hybrides sont synthétisés par simple mélange d'une dispersion aqueuse de microgels P(MEO₂MA-*co*-OEGMA-*co*-MAA) avec une dispersion de nanoparticules de maghémites stabilisée à pH 2 (nanoparticules de charges cationiques). L'encapsulation des nanoparticules au sein des microgels se fait en 2 étapes :
- Une première étape consiste à ajouter les nanoparticules cationiques au sein d'une solution de microgels dispersés à pH 3 et à température ambiante. Ces conditions de mélange permettent de garder la charge cationique à la surface des nanoparticules γ-Fe₂O₃. Les nanoparticules vont préférentiellement interagir avec les microgels grâce aux groupements acides carboxyliques issus des unités acides méthacryliques contenues au sein des microgels. En effet, les groupements acides carboxyliques ont la faculté de s'adsorber à la surface de particules d'oxyde métallique telles que l'oxyde de fer et de plus, la charge positive à la surface des nanoparticules permet une interaction privilégiée. En ce sens, à l'ajout de nanoparticules γ-Fe₂O₃, ces dernières vont préférentiellement se localiser au sein des microgels (étape résumée sur la Figure 4).
- Une seconde étape consiste à augmenter le pH du milieu (microgels + nanoparticules) en partant d'un pH 3 jusque pH 7.5. Cette élévation du pH induit : 1) Une déstabilisation des nanoparticules γ-Fe₂O₃ cationiques au sein du mélange. En effet, les nanoparticules présentant un point de charge nul à pH neutre (Point isoélectrique = 7.2), ces dernières floculent à ce pH par manque de répulsion électrostatique. 2) La création de charges négatives au sein des microgels issus des fonctions acides carboxyliques (COOH) sous forme de groupements carboxylates (COO⁻ ). La concomitance de ces deux phénomènes permet d'ancrer les nanoparticules magnétiques au sein du microgel et d'améliorer la stabilité des microgels hybrides grâce aux charges négatives des fonctions carboxylates (étape résumée en Figure 5).

### Protocole expérimental

Un volume de 40 mL d'une dispersion aqueuse de microgels P(MEO₂MA-*co-*OEGMA-co-MAA) de concentration massique de 1.45 g.L⁻¹ est introduit dans un ballon de 100 mL et laissé sous agitation magnétique, le pH de la dispersion est ajusté à 3.0 par addition d'une solution d'acide nitrique (HNO₃) à 0.1 mol.L⁻¹. Puis, un volume de 10 mL d'une dispersion de nanoparticules de magnétite cationiques à pH 3 de concentration massique 1.34 g.L⁻¹ est ajouté goutte à goutte au mélange à température ambiante et sous agitation magnétique, cela correspond à une quantité de nanoparticules par microgels hybrides de ∼18.8 %. Le mélange réactionnel est laissé sous agitation et à température ambiante durant 12 h. Le pH du mélange réactionnel est alors augmenté par ajout, goutte à goutte, d'une solution d'hydroxyde de potassium (KOH) à 0.5 mol.L⁻¹. Finalement, Les microgels hybrides sont séparés du milieu réactionnel par centrifugation (5 000 rpm, 20 min) et le milieu réactionnel est remplacé par de l'eau pure (de grade milliQ). La solution finale se compose alors d'une dispersion colloïdale de microgels P(MEO₂MA-*co*-OEGMA-*co*-MAA) dans de l'eau, cette dispersion est gardée à température ambiante. Différentes synthèses ont été effectuées en variant la fraction massique théorique de nanoparticules par microgel hybride entre 0 et 33 %.

### Propriété des Microgels hybrides γ-Fe₂O₃ / P(MEO₂MA-co-OEGMA-co-MAA)

Les microgels hybrides ont été caractérisés à l'état sec par microscopie électronique à transmission (MET) et à l'état humide par diffusion dynamique de la lumière. L'architecture hybride des microgels a été démontrée par MET, l'observation des microgels à l'état sec permet de mettre en évidence la bonne encapsulation des nanoparticules magnétiques au sein des microgels qui ne sont pas expulsés au cours du traitement de séchage. Le taux de charge des nanoparticules encapsulées a été déterminé par analyse thermogravimétrique, l'analyse confirme une encapsulation quantitative et importante des nanoparticules (taux de charge testés allant de 0 à 33 % massique de nanoparticules par microgel hybride). Les propriétés thermosensibles des microgels hybrides en solution aqueuse à pH neutre sont également démontrées avec une contraction des microgels hybrides qui passent de 1000 nm à 20 °C à 450 nm avec une température de contraction de 37 °C. Cette contraction à pH neutre s'opère quelques soit le taux de charge en nanoparticules magnétiques.

### EXEMPLE 4 : Films de microgels

On a utilisé les compositions de microgels résumées dans le Tableau 1 pour préparer des films.

**Tableau 1. Composition en monomères des microgels**

| | **Microgels P(MEO₂MA-co-OEGMA-co-MAA)** | | **Microgels hybrides P(MEO₂MA-co-OEGMA-co-MAA)/γ-Fe₂O₃** | | |
|---|---|---|---|---|---|
| Microgel | **1** | **2** | **3** | **4** | **5** |
| **MEO₂MA** (mmol/L) | 88.4 | 83.9 | 83.9 | 83.9 | 83.9 |
| **OEGMA** (mmol/L) | 9.82 | 9.36 | 9.36 | 9.36 | 9.36 |
| **MAA** (mmol/L) | 0 **^{a}** | 4.99 **^{b}** | 4.99 **^{b}** | 4.99 **^{b}** | 4.99 **^{b}** |
| **OEGDA** (mmol/L) | 1.92 | 1.92 | 1.92 | 1.92 | 1.92 |
| **KPS** (mmol/L) | 0.86 | 0.86 | 0.86 | 0.86 | 0.86 |
| **γ-Fe₂O₃** (wt%) | 0 | 0 | 4.7 **^{c}** | 9.1 **^{c}** | 16 **^{c}** |
| **Morphologie des microgels** | 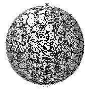 | 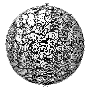 | 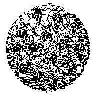 | 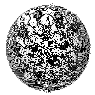 | 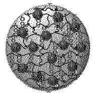 |

| | | | | | |
|---|---|---|---|---|---|
| **^{a} Microgel 1** contient 0 mol% d'unités MAA ; **^{b} Microgels 2, 3, 4** et **5** contiennent 3.5 mol% d'unités MAA incorporés dans le microgel (soit 70 % de monomère MAA initial) ; ^{c}Taux de nanoparticules γ-Fe₂O₃ par microgels hybrides déterminés par analyse thermogravimétrique (TGA). | | | | | |

Les films sont préparés par un procédé de séchage présenté sur la Figure 6. Partant d'une dispersion colloïdale de microgels monodisperses (de taille pouvant varier entre 500 et 1000 µm en solution) à une concentration massique en microgels variant de 1.4 à 5 wt% dans l'eau **(solution 1).** Un volume constant de solution est introduit dans un moule en plastique et laissé sécher jusqu'à complète évaporation de l'eau (Etape 1 de la Figure 6). Le film restant au fond du moule est alors composé de plusieurs couches de microgels monodisperses et totalement sec (de taille pouvant varier entre 350 et 450 µm à l'état sec). Le film est soigneusement récupéré et réintroduit dans une solution aqueuse (**solution 2**). On fait varier différents paramètres: **1.)** La concentration massique en dispersion de 1.4 à 5 wt% permet de faire varier l'épaisseur de film gonflé (fin de l'étape 3 : épaisseur de 200 µm à 1000 µm). **2.)** le pH de **solution 2** est varié entre 5.5 et 7.5.

### Protocole expérimental: Formation des films de microgels à base de poy(oligo-(éthylène glycol) méthacrylate) thermosensibles.

Un volume de 5 mL d'une dispersion colloïdale de microgels à une concentration massique de 1.4 à 5 wt% est introduit dans un moule en plastique et laissé séché à une température de 32°C (+/- 2°C). Après complète évaporation du solvant, le film est soigneusement récupéré puis introduit dans une solution aqueuse et laissé gonfler à température ambiante.

Les films récupérés ont été observés par microscopie à force atomique à l'état sec et caractérisés au rhéomètre à l'état humide (Fin **d'étape 3 de la** **Figure 6**). Les microgels forment un film élastique composés de plusieurs couches de microgels (à **l'étape 2**) et ce quelle que soit la composition des microgels (**microgels 1** à **5** en Tableau 1). A l'inverse, les microgels perdent leurs propriétés mécaniques lorsqu'ils sont gonflés dans l'eau mais ne se re-dispersent pas en solution.

**Microgels 1** et **2** : on a fait varier l'épaisseur de film de 200 à 1000 µm, la multiplication des couches de microgels ne modifie pas le phénomène de « filmification » et les films gardent leur élasticité à l'état sec.

**Microgels 3, 4** et **5** : Une épaisseur de l'ordre de 300 µm (films gonflés à **l'étape 3**) a été étudiée dans le cas des microgels hybrides. L'ajout de nanoparticules ne modifie pas les propriétés filmogènes des microgels. Au contraire, les propriétés mécaniques des films sont grandement améliorées à l'état humide.

### Evaluation des propriétés mécano-électriques des films

### 1. Caractérisation des propriétés mécano-électriques des films

Les propriétés mécano-électriques des films de microgels ont été étudiées. Il s'agit là de démontrer la capacité des microgels à générer un courant électrique lorsque l'on exerce une pression sur ces microgels. Plus particulièrement, l'idée de l'invention est de générer un courant électrique en pressant le matériau à la surface d'un substrat. Ce potentiel électrique peut être généré à partir d'un matériau présentant des fonctions ioniques attachées de manière covalente (ou matériau polyélectrolyte). En effet, les groupements ioniques étant attachés dans le microgel, seuls les contre-ions de chaque groupement carboxylates ont une mobilité dans le microgel. Lorsqu'une pression/déformation unidirectionnelle est exercée sur ces microgels polyélectrolytes, la mobilité des contre-ions est favorisée créant ainsi une polarisation entre les charges positives des contre-ions mobiles et les charges négatives des groupements carboxylates attachés. Ce gradient ionique se traduit par un potentiel électrique à l'interface. Ainsi, la présence de fonctions ionisables dans le microgel permettrait de créer une polarisation au sein du matériau et de générer un potentiel électrique.

Un montage est utilisé afin de mettre en évidence les propriétés mécano-électriques des films. Pour cela, un rhéomètre Anton Paar MCR301 est utilisé en géométrie plan-plan au sein duquel deux électrodes plates et conductrices à base d'Indium Tin Oxide ou ITO (**entité 1.** de la Figure 7) sont accrochées de part et d'autre de la géométrie. L'électrode inférieure est fixe et l'électrode supérieure est amovible. Un film humide de microgels (**entité 3.** de la Figure 7) est déposé sur la surface de l'électrode inférieure et l'électrode supérieure est abaissée afin d'exercer une compression de force F_{N} sur le film. En contrôlant la distance entre les deux électrodes, il est possible de contrôler la force d'écrasement (F_{N}) exercée sur le film.

Un programme de compression/relaxation est effectué afin de faire varier la force exercée sur le film. Dans un premier temps, l'épaisseur initiale de film humide (notée L₀) a été déterminée et la distance entre les deux électrodes a été progressivement diminuée d'une distance ΔL en abaissant l'électrode supérieure. Le programme se distingue par une période courte d'écrasement (τ= 2 secondes) avec une distance finale L puis une période longue de relaxation (τ= 20 secondes) avec un retour à l'état initial L₀, le tout permet de simuler une action de type « touch-sensitive » sur le film (Figure 8).

A la période d'écrasement, une force normale F_{N} (en newton) est enregistrée. Cette force F_{N} est proportionnelle à l'épaisseur d'écrasement (ΔL). Le programme se compose comme tel : le film est compressé à jusqu'à une distance L= L₀-ΔL avec ΔL= (3x) 10%.L₀, puis (3x) 20%.L₀, puis (3x) 25%.L₀ etc. Un exemple de programme utilisé est donné en Figure 9.

Enfin, les électrodes supérieures et inférieures sont reliées à un convertisseur/amplificateur, afin d'enregistrer la différence de potentiel (notés E) générée entre les deux électrodes tout au long du programme.

### 2. Etude des Films de microgels

### Films de microgels P(MEO₂MA-co-OEGMA-co-MAA)

Les films de microgels biocompatibles P(MEO₂MA-*co*-OEGMA-*co*-MAA) ont été caractérisés en faisant varier 3 paramètres :
1.) L'effet de la multiplication des compressions : pour chaque films, une compression de même force est répétée successivement (3 fois comme représenté en Figure 9) et le potentiel de chaque compression est analysé.
2.) Epaisseur de films : deux épaisseurs de films ont été testées afin de déterminer l'impact de l'épaisseur sur la capacité des films à générer un potentiel électrique à l'interface (∼200 µm et ∼900-1000 µm).
3.) Composition en fonctions acide carboxyliques : la composition en unité MAA a été variée de 0 à 3.5 mol% en MAA (**microgels 1** et **2**) afin d'évaluer l'impact des unités MAA sur le potentiel électrique.
4.) Le pH de solution dans lequel les films sont gonflés : le pH permet de varier la quantité de fonction ioniques (COO⁻) au sein du microgel. En effet les fonctions acides carboxyliques sont présentes sous forme de deux espèces protonée (COOH) et déprotonée ou ionisé (COO⁻). La proportion de ces deux espèces dépend du pH de la solution avec une augmentation de l'espèce ionisée COO⁻ lorsque l'on augmente le pH (pH 5.5 →%COO⁻= 0 ; pH 6.5 →%COO⁻= 50 % ; pH 7.5 →%COO⁻= 75%).

**Tableau 2. Récapitulatif des échantillons étudiés.**

| **Effet de l'épaisseur de film** | | | | | | |
|---|---|---|---|---|---|---|
| **Echantillon** | **MAA (mol%)** | **γ-Fe₂O₃ (wt%)** | **Epaisseur de film gonflé (µm)** | **pH** | **Eₘₐₓ (mV)** | **F_{N} (N)** |
| **Microgel 2** | 3.5 | 0 | 180 | 6.5 | 5.4 - 11.1 | 0.38 - 0.40 |
| **Microgel 2** | 3.5 | 0 | 850 | 6.5 | 2.0 - 5.6 | 0.30 - 0.35 |

| **Effet de la composition en MAA** | | | | | | |
|---|---|---|---|---|---|---|
| **Echantillon** | **MAA (mol%)** | **γ-Fe₂O₃ (wt%)** | **Epaisseur de film gonflé (µm)** | **pH** | **Eₘₐₓ (mV)** | **F_{N} (N)** |
| **Microgel 1** | **0** | 0 | **275** | 6.5 | 0.43 - 0.9 | 0.30 - 0.35 |
| **Microgel 2** | **3.5** | 0 | **180** | 6.5 | 5.4 - 11.1 | 0.38 - 0.40 |

| **Effet du pH** | | | | | | |
|---|---|---|---|---|---|---|
| **Echantillon** | **MAA (mol%)** | **γ-Fe₂O₃ (wt%)** | **Epaisseur de film gonflé (µm)** | **pH** | **Eₘₐₓ (mV)** | **F_{N} (N)** |
| **Microgel 2** | 3.5 | 0 | 310 | **5.5** | 2.0-9.9 | 0.7 - 0.9 |
| **Microgel 2** | 3.5 | 0 | 180 | **6.5** | 5.4 - 11.1 | 0.38 - 0.40 |
| **Microgel 2** | 3.5 | 0 | 350 | **7.5** | 5.5 - 11.2 | 0.18 - 0.23 |

| **Effet de γ-Fe₂O₃** | | | | | | |
|---|---|---|---|---|---|---|
| **Echantillon** | **MAA (mol%)** | **γ-Fe₂O₃ (wt%)** | **Epaisseur de film gonflé (µm)** | **pH** | **Eₘₐₓ (mV)** | **F_{N} (N)** |
| **Microgel 2** | 3.5 | **0** | 180 | 6.5 | 5.4 - 11.1 | 0.38 - 0.40 |
| **Microgel 2** | 3.5 | **4.7** | 310 | 6.5 | 8.7 - 12.2 | 0.65 - 1.13 |
| **Microgel 2** | 3.5 | **9.1** | 320 | 6.5 | 2.4 - 3.7 | 1.0 - 1.2 |
| **Microgel 2** | 3.5 | **16** | 180 | 6.5 | 3.7 - 3.8 | 3.0 - 3.1 |

### Résultats :

En observant l'évolution du signal électrique au cours de la compression des films de microgels, un effet mécano-électrique est mis en évidence sur l'ensemble des films caractérisées. Cet effet mécano-électrique est fonction de la force F_{N} exercée sur les films avec un potentiel électrique qui augmente avec la force de compression. De plus, une tendance semble se dégager en fonction des paramètres d'analyse :
- Effet de la répétition des compressions : le potentiel électrique enregistré est très important lors de la première compression. Alors que lors de la 2^{ème} et 3^{ème} compression, le potentiel généré est plus faible. Cette première constatation peut être due à un mouvement important des ions à la première compression du film créant un potentiel électrique instantané important (∼ 12 mV). Après un temps de relaxation de 20 s, les compressions suivantes de même force ne semblent pas suffisantes pour provoquer ce même mouvement des ions avec un potentiel généré qui diminue.
- Effet de l'épaisseur de film : le potentiel électrique généré par la compression des films d'épaisseurs différentes montre un faible effet mécano-électrique lorsque le film est trop épais (E = 2 - 5.6 mV pour F_{N,max} = 0.35 N). A l'inverse, un effet mécano-électrique plus important est visible lorsque l'épaisseur de film est faible allant de 11 à 5 mV pour des forces F_{N} de à 0,38 0,4 N. Une épaisseur trop importante ne permettrait donc pas d'impacter suffisamment sur la mobilité des ions. (Cf. Tableau 2. *Effet de l'épaisseur de film*)
- Composition en fonctions acides carboxyliques : la présence d'acide méthacrylique semble améliorer les propriétés mécano-électriques des films. En effet, les films semblent plus sensibles aux effets de compression avec un potentiel électrique mesuré pour des forces faibles (11 - 5 mV à 0.4 N avec MAA contre 1 - 0.5 mV à 0.4 N sans MAA). De plus, la mesure effectuée à pH 6.5 met en avant l'importance des groupements carboxylates ionisés issus des unités MAA (50 % de groupement MAA ionisé) sur la sensibilité des films de microgels. (Cf. Tableau 2. *Effet de la composition en MAA)*
- Le pH de la solution de 5.5 à 7.5 sur les films de microgels de même composition ne semble pas modifier la valeur de potentiel électrique des films mais lorsque le pH de solution est augmenté, la perte du potentiel électrique face aux répétitions de compression semble ralentie. En effet à pH 5.5, la répétition de la compression fait chuter le potentiel électrique jusqu'à 2 mV alors qu'à un pH plus élevé, le potentiel chute jusqu'à 5.4 mV Ceci est probablement dû à l'augmentation de la proportion des fonctions carboxylate ionisées (%COO_{pH5.5} = 0 % ; %COO_{pH6.5} = 50 %; %COO_{pH 7.5} = 75 %), augmentant la capacité de polarisation des microgels qui composent le film. Les films sont alors plus sensibles lorsque le pH est augmenté. (Cf. Tableau 2. *Effet du pH)*

### Films de microgels hybrides P(MEO₂MA-co-OEGMA-co-MAA) / γ-Fe₂O₃

Les films de microgels hybrides ont été caractérisés à pH 7.5 et ont été comparés à un film de microgels sans nanoparticules (NPs) magnétiques. Un film de microgels sans nanoparticules présente un potentiel maximum de 6 mV pour une force de compression F_{N} = 0.4 N. Pour les films de microgels avec nanoparticules, le potentiel généré dépend de la quantité de nanoparticules incorporées :
- Pour ∼5 wt% de nanoparticules magnétiques incorporées, les nanoparticules n'ont pas d'effet sur le potentiel électrique généré et la réponse du film aux compressions est de 6-7 mV.
- Pour 9 et 17 wt% de nanoparticules magnétiques incorporées, une diminution du potentiel électrique, qui atteint 2.5 mV quelle que soit la force de compression est observée. Cette perte de potentiel électrique peut être attribuée à la diminution de charges issues des unités acides carboxyliques à pH 7.5 car elles interagissent déjà avec les NPs. En effet, l'incorporation des nanoparticules se fait par adsorption de ces dernières au niveau des sites ioniques (COO⁻) contenus dans les microgels. Cette adsorption semble diminuer la fraction de sites ioniques encore disponibles au sein même du microgel et ainsi diminuer la capacité de polarisation des microgels. Une quantité de 5 wt% en nanoparticules incorporées n'influence pas le comportement polyelectrolyte des films hybrides. (Cf. Tableau 2. *Effet du γ-Fe₂O₃*).

### Propriétés optiques des microgels

Outre les propriétés mécano-électriques, les films de microgels se distinguent par leurs propriétés optiques, liées à la capacité de ces films à diffracter la lumière. Une disparité est observée en fonction de la composition des films :

### Films microgels P(MEO₂MA-co-OEGMA-co-MAA)

Lors du séchage d'une dispersion colloïdale de microgels sans nanoparticules, les films formés sont transparents à l'état sec et iridescent à l'état humide (Figure 10). Il semble que lors du gonflement des microgels le diamètre et la distance entre les particules favorisent une diffraction de la lumière dans le visible, cette diffraction est mise en évidence par l'observation de cristaux photoniques.

### Films microgels hybrides P(MEO₂MA-co-OEGMA-co-MAA) / γ-Fe₂O₃.

Lors du séchage d'une dispersion colloïdale de microgels, on obtient un film transparent et coloré à l'état sec qui présente des propriétés iridescentes en réflexion à des angles d'observation très faibles. Le matériau est alors marron (couleur dûe aux nanoparticules magnétiques) en observant à 90° et iridescent en observant à des angles plus faibles (Figure 11).

Les propriétés photoniques sont visibles notamment en réflexion (sur fond sombre) et très peu en transmission (sur fond clair) comme observés en Figure 12.

### Propriétés mécaniques et magnétiques des films hybrides

Outre les propriétés de filmification des microgels hybrides et leurs propriétés optiques l'ajout de nanoparticules magnétiques permet également d'orienter les microgels au cours du séchage. La Figure 13 illustre bien ces propriétés puisqu'il est possible de concentrer les microgels au cours du séchage en un point précis par application d'un champ magnétique (dans notre cas l'aimant a été posé en-dessous de la dispersion). Le séchage permet d'une part, de figer le tout en un point ciblé et d'autre part, de modifier la teinte du film final par une concentration localisée des microgels hybrides tout en gardant les propriétés iridescentes en réflexion (*Solution séchée avec aimant,* Figure 13).

## Revendications

1. Procédé de soin ou de maquillage cosmétique qui consiste à appliquer sur la peau des microgels ou un produit cosmétique contenant lesdits microgels,
les microgels étant susceptibles d'être obtenus par polymérisation par précipitation en phase aqueuse en présence d'un agent réticulant, des trois monomères suivants :
- le di(éthylène glycol) méthyl éther méthacrylate (M(EO)₂MA),
- un oligo(éthylène glycol) méthyl éther méthacrylate (M(EO)ₙMA) n étant un entier allant 3 à 12,
- un monomère de formule CR₁R₂=CR₃R₄ dans laquelle R₁, R₂, R₃ et R₄ représentent un hydrogène, un halogène ou un groupement hydrocarboné, à la condition qu'au moins un des quatre groupements comprenne un groupement -COOH ou -COO⁻M⁺, tel que M⁺ représente un cation,
et ledit produit cosmétique contenant, outre les microgels, au moins un composé choisi dans le groupe constitué par les agents tensio-actifs, les huiles, les produits biologiquement actifs, les pigments et les colorants.

2. Procédé selon la revendication précédente, **caractérisé en ce que** M(EO)₂MA représente 50% à 90 % en moles du nombre total de moles des trois monomères, M(EO)ₙMA représente 10 à 50% en moles du nombre total de moles des trois monomères et le monomère de formule CR₁R₂=CR₃R₄ représente 0,1 à 20 % en moles du nombre total de moles des trois monomères.

3. Procédé selon l'une des revendications précédentes, **caractérisé en ce que** le monomère de formule CR₁R₂=CR₃R₄ est l'acide méthacrylique.

4. Procédé selon l'une des revendications précédentes, **caractérisé en ce que** l'agent de réticulation est un oligo(éthylène glycol) diacrylate (OEGDA) comprenant de 1 à 10 motifs éthylène glycol.

5. Procédé selon l'une des revendications précédentes, **caractérisé en ce que** les microgels comprennent des nanoparticules métalliques ou d'oxydes métalliques.

## Patentansprüche

1. Verfahren zur kosmetischen Pflege oder zum Schminken, das darin besteht, Mikrogele oder ein kosmetisches Produkt, das diese Mikrogele enthält, auf die Haut aufzutragen,
wobei die Mikrogele durch wässrige Phasenausfällungspolymerisation in Gegenwart eines Vernetzungsmittels der folgenden drei Monomere erhältlich sind:
- Di(ethylenglykol)methylether-methacrylat (M(EO)₂MA),
- Oligo(ethylenglykol)methylether-methacrylat (M(EO)ₙMA), wobei n eine ganze Zahl von 3 bis 12 ist,
- ein Monomer der Formel CR₁ R₂=CR₃R₄, wobei R₁, R₂, R₃ und R₄ Wasserstoff, Halogen oder eine Kohlenwasserstoffgruppe darstellen, vorausgesetzt, dass mindestens eine der vier Gruppen eine -COOH- oder -COO⁻M⁺-Gruppe umfasst, so dass M⁺ ein Kation darstellt,
und wobei das kosmetische Produkt neben Mikrogelen mindestens eine Verbindung enthält, ausgewählt aus der Gruppe bestehend aus Tensiden, Ölen, biologisch aktiven Produkten, Pigmenten und Farbstoffen.

2. Verfahren nach dem vorstehenden Anspruch, **dadurch gekennzeichnet, dass** M(EO)₂MA 50 bis 90 Mol-% der Gesamtmolzahl der drei Monomere darstellt, M(EO)ₙMA 10 bis 50 Mol-% der Gesamtmolzahl der drei Monomere darstellt und das Monomer der Formel CR₁R₂=CR₃R₄ 0,1 bis 20 Mol-% der Gesamtmolzahl der drei Monomere darstellt.

3. Verfahren nach einem der vorstehenden Ansprüche, **dadurch gekennzeichnet, dass** es sich bei dem Monomer der Formel CR₁ R₂=CR₃R₄ um Methacrylsäure handelt.

4. Verfahren nach einem der vorstehenden Ansprüche, **dadurch gekennzeichnet, dass** es sich bei dem Vernetzungsmittel um ein Oligo(ethylenglykol)diacrylat (OEGDA) mit 1 bis 10 Ethylenglykoleinheiten handelt.

5. Verfahren nach einem der vorstehenden Ansprüche, **dadurch gekennzeichnet, dass** die Mikrogele metallische Nanopartikel oder Metalloxide umfassen.

## Claims

1. A cosmetic makeup or care process that consists in applying to the skin microgels or a cosmetic product comprising said microgels,
said microgels being obtainable by aqueous phase precipitation polymerization of the following three monomers in the presence of a crosslinking agent:
- di(ethylene glycol) methyl ether methacrylate (M(EO)₂MA),
- an oligo(ethylene glycol) methyl ether methacrylate (M(EO)ₙMA) n being an integer ranging from 3 to 12,
- a monomer of formula CR₁R₂=CR₃R₄ in which R₁, R₂, R₃ and R₄ represent a hydrogen, a halogen or a hydrocarbon group, on condition that at least one of the four groups comprises a -COOH or -COO⁻M⁺ group, M⁺ representing a cation,
said cosmetic product comprising, besides microgels, at least one compound chosen from the group consisting of surfactants, oils, biologically active products, pigments and dyes.

2. The process as claimed in the preceding claim, **characterized in that** M(EO)₂MA represents 50 mol% to 90 mol% of the total number of moles of the three monomers, M(EO)ₙMA represents 10 mol% to 50 mol% of the total number of moles of the three monomers and the monomer of formula CR₁R₂=CR₃R₄ represents 0.1 mol% to 20 mol% of the total number of moles of the three monomers.

3. The process as claimed in one of the preceding claims, **characterized in that** the monomer of formula CR₁R₂=CR₃R₄ is methacrylic acid.

4. The process as claimed in one of the preceding claims, **characterized in that** the crosslinking agent is an oligo(ethylene glycol) diacrylate (OEGDA) comprising from 1 to 10 ethylene glycol units.

5. The process as claimed in one of the preceding claims, **characterized in that** microgels comprise metal or metal oxide nanoparticles.
